# EUROPEAN PATENT APPLICATION

(11) **EP 0 903 147 A2**
(43) Date of publication of application: **24.03.1999**
(21) Application number: 98307522.7
(22) Date of filing: 16.09.1998
(51) Int. Cl.: A61K 38/17, A61K 38/18, C12N 5/00

(54) **Use of REG-2 as a Schwann cell mitogen**

(30) Priority: 17.09.1997 GB 9719796
(71) Applicant: MEDICAL RESEARCH COUNCIL, London W1N 4AL (GB)
(72) Inventor: Livesey, Frederick John, Blackrock, Co. Dublin (IE); Hunt, Stephen Philip, Cambridge CB4 3BE (GB)
(74) Representative: Harding, Charles Thomas

(57) **Abstract**

A Schwann cell mitogen that is capable of being upregulated during regeneration of neuronal cells or tissue is described. In a particular embodiment, there is provided the use of a protein comprising the sequence presented as SEQ ID No. 1 or a variant, derivative or homologue thereof in the manufacture of a medicament to act as a Schwann cell mitogen.

### Annex to the description

SEQUENCE LISTING

<110> Medical Research Council

<120> Medicament

<130> Application number: 98307522.7

<140>
<141>

<160> 3

<170> PatentIn Ver. 2.0

<210> 1
<211> 175
<212> PRT
<213> mammalian

<400> 1
Met Leu His Arg Leu Ala Phe Pro Val Met Ser Trp Met Leu Leu Ser
1               5                   10                  15

Cys Leu Met Leu Leu Ser Gln Val Gln Gly Glu Asp Ser Pro Lys Lys
            20                  25                  30

Ile Pro Ser Ala Arg Ile Ser Cys Pro Lys Gly Ser Gln Ala Tyr Gly
        35                  40                  45

Ser Tyr Cys Tyr Ala Leu Phe Gln Ile Pro Gln Thr Trp Phe Asp Ala
    50                  55                  60

Glu Leu Ala Cys Gln Lys Arg Pro Glu Gly His Leu Val Ser Val Leu
65                  70                  75                  80

Asn Val Ala Glu Ala Ser Phe Leu Ala Ser Met Val Lys Asn Thr Gly
                85                  90                  95

Asn Ser Tyr Gln Tyr Thr Trp Ile Gly Leu His Asp Pro Thr Leu Gly
            100                 105                 110

Gly Glu Pro Asn Gly Gly Gly Trp Glu Trp Ser Asn Asn Asp Ile Met
        115                 120                 125

Asn Tyr Val Asn Trp Glu Arg Asn Pro Ser Thr Ala Leu Asp Arg Gly
    130                 135                 140

Phe Cys Gly Ser Leu Ser Arg Ser Ser Gly Phe Leu Arg Trp Arg Asp
145                 150                 155                 160

Thr Thr Cys Glu Val Lys Leu Pro Tyr Val Cys Lys Phe Thr Gly
                165                 170                 175

<210> 2
<211> 781
<212> DNA
<213> mammalian

<400> 2
aaaaccatcc aaatcgcccg caagacagct aaggaggagc agaaagatga tgagagttaa 60
tatgttgcat cgcttggcct tcccagtcat gtcctggatg ctgctctcct gcctgatgct 120
cttatcacag gtgcaaggag aagactctcc gaagaaaata ccctctgcac gcattagttg 180
ccccaaaggc tcccaggcat atggctccta ctgctatgcc ctgtttcaga taccacagac 240
ctggtttgat gcagaactgg cctgccagaa gagacctgaa ggacaccttg tatctgtgct 300
caatgtagct gaagcttcat tcttggcatc catggtcaag aacactggaa acagctacca 360
atatacctgg attggactcc atgaccccac tcttggtgga gaacccaatg gaggtggatg 420
ggagtggagt aacaatgaca taatgaatta tgtcaactgg gagaggaacc catctactgc 480
cttagaccgc ggattctgtg gcagcttgtc aagatcttct ggatttctaa gatggagaga 540
taccacatgt gaagtgaagt tgccctacgt ctgcaaattt acaggttaaa attaccagac 600
agcaaacagc tttagtttgt cctgaagcac atcctgtcaa ggggcaaaat atgaagactt 660
gcgtagaaaa agtgtattct atctacagtc catattggag ctctaatcat tctttagcca 720
attttgtata agttgtgtcc tcatgtcttg gaaagcagta ataaacctca gtctctcttc 780
g                                                                  781

<210> 3
<211> 45
<212> DNA
<213> mammalian

<400> 3
tgggagcctt tggggcaact aatgcgtgca gagggtattt tcttc                  45

## Description

The present invention relates to a medicament. In particular, the present invention relates to a medicament that can be used to affect the development (e.g. repair and/or regeneration) of neuronal cells or tissue.

Motor neurons are the only adult mammalian central neurons that have the capability to regenerate following injury¹. This ability is dependent on the environment of the peripheral nerve and an intrinsic capacity of motor neurons for regrowth².

For example, Schwann cell dedifferentiation and proliferation are considered to be essential for neural regeneration in the adult peripheral nervous system^{5, 6}. The currently known Schwann cell mitogens, such as the glial growth factors/neuregulins, are consitutively expressed by motor and sensory neurons in early development and adulthood²⁴ but are not upregulated during regeneration²⁵.

However, regeneration of neuronal tissue is by no means guaranteed following injury and so it is desirable to have compounds and compositions that could be used for the beneficial treatment of damaged neuronal cells or tissue.

The present invention seeks to provide compounds and compositions useful for the beneficial treatment of damaged neuronal cells or tissues.

According to a first aspect of the present invention there is provided the use of a protein comprising the sequence presented as SEQ ID No. 1 or a variant, derivative or homologue thereof in the manufacture of a medicament to act as a Schwann cell mitogen.

According to a second aspect of the present invention there is provided a medicament comprising a protein comprising the sequence presented as SEQ ID No. 1 or a variant, derivative or homologue thereof optionally admixed with a pharmaceutically acceptable carrier, diluent or excipient.

According to a third aspect of the present invention there is provided a medicament comprising a protein comprising the sequence presented as SEQ ID No. 1 or a variant, derivative or homologue thereof optionally admixed with a pharmaceutically acceptable carrier, diluent or excipient, and wherein the protein is in an effective amount to act as a Schwann cell mitogen.

According to a fourth aspect of the present invention there is provided a method of treatment comprising administering to a subject a protein comprising the sequence presented as SEQ ID No. 1 or a variant, derivative or homologue thereof and wherein the protein affects the development (e.g. repair and/or regeneration) a neuronal cell or tissue.

According to a fifth aspect of the present invention there is provided a method of treatment comprising administering to a subject a protein comprising the sequence presented as SEQ ID No. 1 or a variant, derivative or homologue thereof and wherein the protein acts as a Schwann cell mitogen.

According to a sixth aspect of the present invention there is provided a method of treatment comprising administering to a subject a protein comprising the sequence presented as SEQ ID No. 1 or a variant, derivative or homologue thereof and wherein the protein is in an amount to cause regeneration of at least some neuronal cells or tissue.

According to a seventh aspect of the present invention there is provided a process of repairing and/or regenerating a neuronal cell or tissue, the process comprising contacting a damaged neuronal cell or tissue with a protein comprising the sequence presented as SEQ ID No. 1 or a variant, derivative or homologue thereof, and allowing repair and/or regeneration of at least some of the neuronal cell or tissue.

Other aspects of the present invention include medicaments comprising the protein of the present invention that are suitable for use in the above-mentioned processes and methods.

In a broader sense, the present invention also provides a Schwann cell mitogen that is capable of being upregulated during regeneration of neuronal cells or tissue.

The protein shown as SEQ ID No. 1 is sometimes called Reg-2.

One of the advantages of the present invention is that we have now shown that Reg-2 can act as a Schwann cell mitogen - which in turn means that a damaged neuronal cell or tissue can be repaired (at least partially repaired) and/or regenerated.

A further advantage of the present invention is that we have also found that Reg-2 is upregulated. This is advantageous in that administration of Reg-2 to induce neuronal cell or tissue repair may result in expression of endogenous Reg-2, possibly leading to even further neuronal cell or tissue repair.

In a highly preferred embodiment the protein of the present invention is not the naturally occuring protein - i.e. it is not the naturally occurring protein in its natural environment and when expressed by endogenous coding sequences in their natural environment under the control of their naturally associated regulatory elements which are also in their natural environment.

Thus, in a highly preferred embodiment the protein of the present invention is an exogenous protein.

In a preferred embodiment, the protein has the sequence presented as SEQ ID No. 1.

Hence, preferred emdodiments of the present invention are:
i. Use of a protein having the sequence presented as SEQ ID No. 1 thereof in the manufacture of a medicament to act as a Schwann cell mitogen.
ii. A medicament comprising a protein having the sequence presented as SEQ ID No. 1 optionally admixed with a pharmaceutically acceptable carrier, diluent or excipient.
iii. A medicament comprising a protein having the sequence presented as SEQ ID No. 1 optionally admixed with a pharmaceutically acceptable carrier, diluent or excipient, and wherein the protein is in an effective amount to act as a Schwann cell mitogen.
iv. A method of treatment comprising administering to a subject a protein having the sequence presented as SEQ ID No. 1 and wherein the protein affects the development (e.g. repair and/or regeneration) of neuronal cell or tissue.
v. A method of treatment comprising administering to a subject a protein having the sequence presented as SEQ ID No. 1 and wherein the protein acts as a Schwann cell mitogen.
vi. A method of treatment comprising administering to a subject a protein having the sequence presented as SEQ ID No. 1 and wherein the protein is in an amount to cause regeneration of at least some neuronal cells or tissue.
vii. A process of repairing and/or regenerating a neuronal cell or tissue, the process comprising contacting a damaged neuronal cell or tissue with a protein having the sequence presented as SEQ ID No. 1 and allowing repair and/or regeneration of at least some of the neuronal cell or tissue.

The protein may be isolated from natural sources or it may be prepared synthetically. In a preferred embodiment, it is prepared by use of recombinant DNA techniques - such as by expression of the nucleotide sequence presented as SEQ ID No. 2 by a suitable expression system.

In the process of the present invention, some or all of the neuronal cells or tissue may be repaired and/or regenerated (or combinations thereof) *in vitro* (i.e. *ex vivo -* namely not in a whole living organism) and then optionally introduced into a subject.

Alternatively, in the process of the present invention, the neuronal cell or tissue may be repaired and/or regenerated (or combinations thereof) *in vivo* in a subject.

Preferably, the neuronal cell or tissue is from or is in an adult animal.

More preferably, the neuronal cell or tissue is from or in an adult human.

Preferably the neuronal cell or tissue is a motor neuronal cell or tissue.

The term "subject" includes any one or more different types of animal, including humans. Preferably, the term means a human.

The term "variant, derivative or homologue" in relation to the protein of the present invention includes any substitution of, variation of, modification of, replacement of, deletion of or addition of one (or more) amino acid from or to the sequence providing the resultant amino acid sequence has the capability to act as a Schwann cell mitogen, preferably having at least the same activity as the enzyme shown as SEQ I.D. No. 1. In particular, the term "homologue" covers homology with respect to structure and/or sequence and/or function. With respect to sequence homology (i.e. similarity), preferably there is at least 75%, more preferably at least 85%, more preferably at least 90% homology to the sequence shown as SEQ ID No.1 in the attached sequence listings. More preferably there is at least 95%, such as at least 98%, homology to the sequence shown as SEQ ID No. 1 in the attached sequence listings.

The term thus also includes active fragments of the protein - but wherein those fragments are also capable of acting as a Schwann cell mitogen.

The term "Schwann cell mitogen" means that the protein of the present invention may independently act as a mitogen of Schwann cells or it may act together with one or more other co-factors (such as a receptor) as a mitogen of Schwann cells.

Thus in accordance with a preferred aspect of the present invention we have found that it is possible to regenerate motor neurons by use of a novel Schwann cell mitogen. In this regard, we have found that the protein of the present invention may act as a Schwann cell mitogen.

The present invention will now be described only by way of example, in which reference is made to the following Figures:
Figure 1 which shows photograph images;
Figure 2 which shows photograph images;
Figure 3 which shows a diagram and two graphs;
Figure 4 which shows two graphs; and
Figure 5 which shows photograph images.

### EXPERIMENTAL

### METHODS

### In vivo regeneration studies

All surgery was carried out according to U.K. Home Office guidelines for the ethical treatment of animals. Sciatic nerve crush, tissue fixation and tissue recovery were carried out as described in the art²⁶. Leukaemia inhibitory factor receptor ("LIFR"), mutant and wild-type mouse embryos were bred and genotyped as described in the art⁷. Regenerating motor neurons were retrogradely labelled by injection of Fluorogold (5 µl of a 7% solution in PBS) into the proximal stump of the sciatic nerve following axotomy. Intraneural injection of either pre-immune or anti-Reg-2 antisera was carried out immediately before sciatic nerve crush by injection of 5µl of polyclonal antiserum, raised in rabbit against the whole recombinant protein, into the crush site. Four days later, animals (n=4 for each treatment) were sacrificed and fixed as described above. Tissue from each experiment was coded prior to sectioning and all counts were performed blind.

### mRNA differential display

Differential display PCR on total cellular RNA extracted from control and regenerating dorsal root ganglia was carried out as described in the art³. The sequences of cloned cDNAs were compared to the GenBank non-redundant and EST databases using the BLAST algorithm²⁷.

### In situ hybridisation

Oligonucleotides 45 bases in length complementary to the sequence of rat Reg-2 isolated in this screen were synthesised in-house at the LMB core facility. The sequence was:

TGG GAG CCT TTG GGG CAA CTA ATG CGT GCA GAG GGT ATT TTC TTC.

The oligonucleotide was 3'-tailed with 35S-α-dATP and hybridised to 15µm sections of developing, lesioned and adult tissues as described²⁸.

### Immunohistochemistry

Polyclonal antisera or monoclonal antibodies to Reg-2, CGRP (Affiniti), S100 (Dako) and BrdU (Boehringer) were applied to tissue sections or fixed cells at 4°C for 48 hours. Antibody detection and visualisation were carried out as described in the art²⁹. Controls for each antibody consisted of a preabsorption with recombinant Reg-2 for the Reg-2 polyclonal antiserum and omission of the first antibody or antiserum from the process for all others. For the *in vivo* Reg-2 inhibition experiments, a control experiment to detect any residual anti-Reg-2 antiserum within the tissue from the original procedure was carried out by omitting the Reg-2 polyclonal antiserum and directly applying the appropriate secondary antibody and carrying out the standard detection procedures.

### Schwann cell culture

Primary cultures of dissociated Schwann cells were prepared from the sciatic nerve of neonatal rats and maintained in Dulbecco's minimal essential medium plus N2 supplement as described in the art²⁹. Basic FGF (50 ng/ml; Sigma), recombinant Reg-2 (at a range of concentrations from 2-100 ng/ml; produced as described in the art³⁰) and forskolin (10µm; Sigma) were added to cultures 24 hours after plating, along with 10µm romodeoxyuridine (BrdU) = 24 hours later, cultures were fixed and the cells were processed for BrdU and S100 immunohistochemistry. The proportion of S100-positive cells which were also BrdU-positive was calculated in three separate experiments. The capacity of the anti-Reg-2 antiserum to inhibit Reg-2 signalling *in vitro* was assessed by measuring the ability of a range of concentrations of antiserum to attenuate the mitogenic effect on Schwann cells of a fixed dose of Reg-2 (30 ng/ml).

### RESULTS

The results are presented in the attached Figures, details on which are as follows.

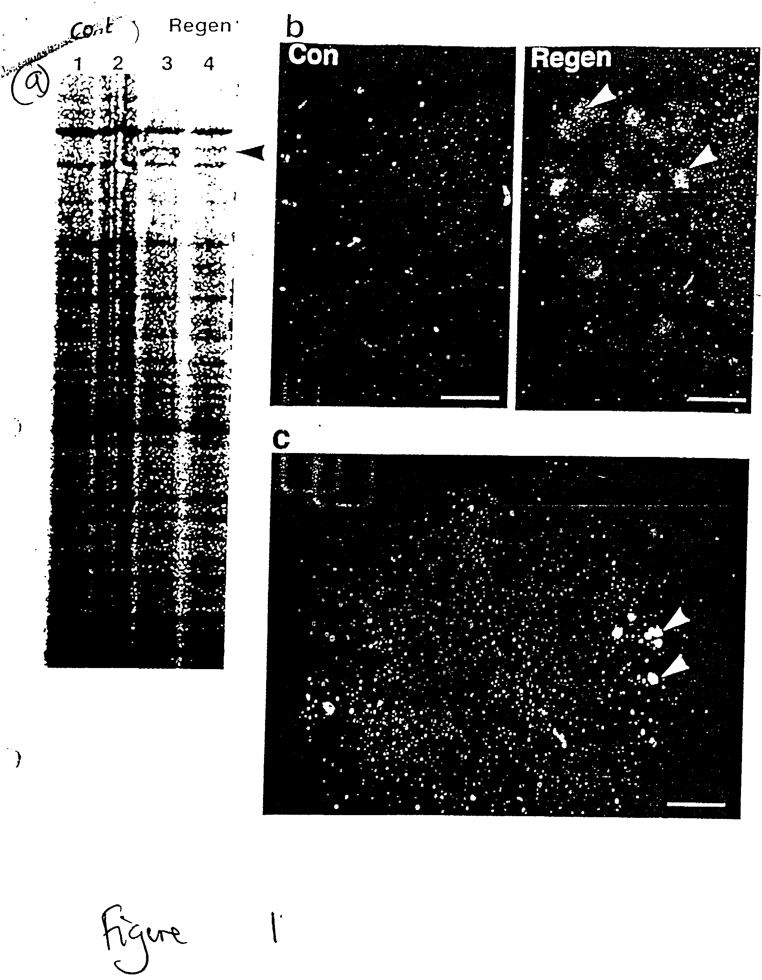

**Figure 1**. Figure 1a, mRNA differential display gel comparing gene expression in resting dorsal root ganglia (DRGs; Control, lanes 1 and 2) with that in regenerating DRGs (Regen, lanes 3 and 4). Arrow indicates a 600 bp cDNA expressed solely in regenerating DRGs which encodes Reg-2. b, confirmation by in situ hybridisation of the induction of Reg-2 expression in regenerating sensory neurons. Arrows indicate Reg-2 expressing neurons. c, Reg-2 expression in spinal cord motor neurons projecting into the regenerating sciatic nerve (arrows) in a coronal section of the lumbar spinal cord. Scale bars: b, 100µm; c, 200µm.

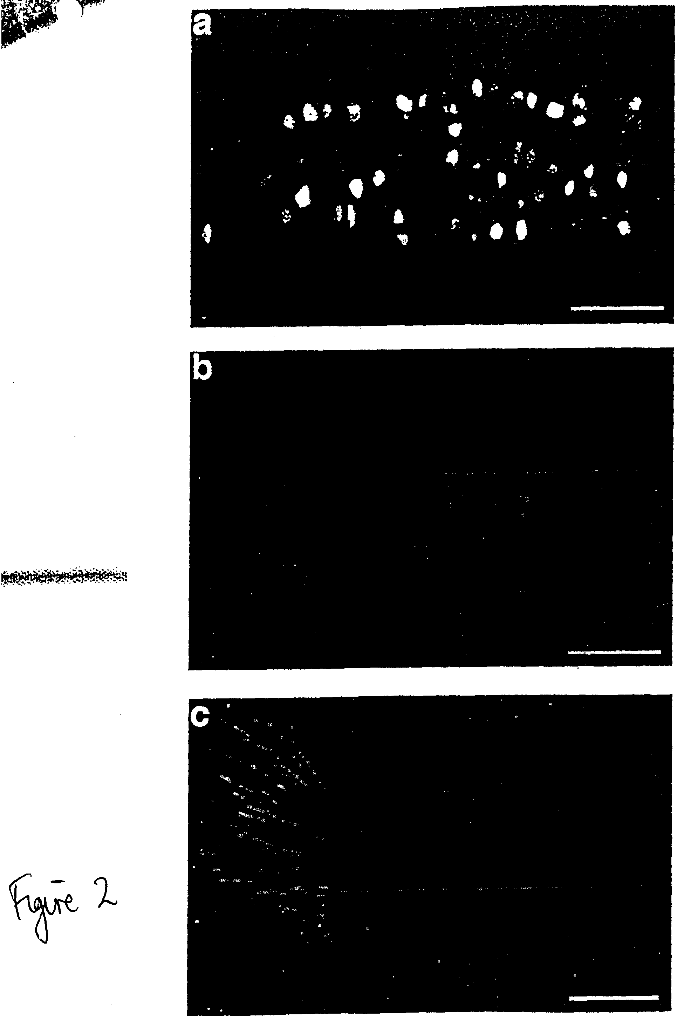

**Figure 2.** Reg-2 protein localisation in regenerating motor neurons. a, longitudinal section through the pool of regenerating sciatic nerve motor neurons, retogradely labelled with Fluorogold. b, fluorescent micrograph of Reg-2 immunoreactivity in the same section. Almost all regenerating motor neurons express Reg-2. c, Reg-2 is orthogradely transported along regenerating axons in the peripheral nerve, as demonstrated by Reg-2 immunoreactivity in the peripheral axons proximal, but not distal, to the site of peripheral nerve ligation. Scale bars in all figures 250µm.

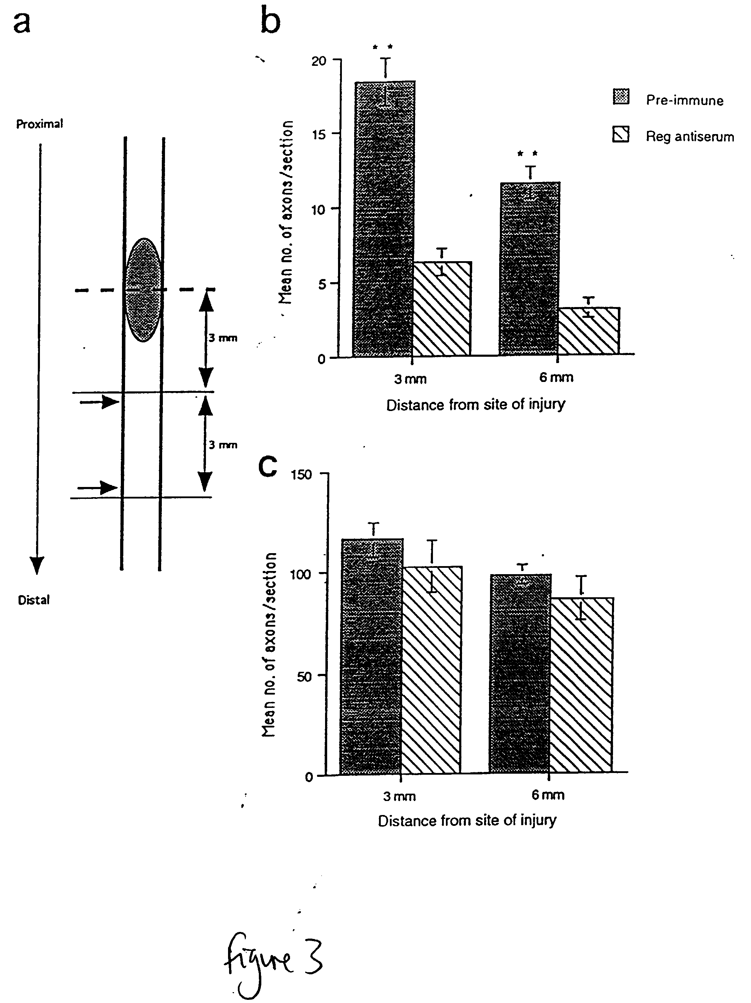

**Figure 3**. Figure 3a, Experimental procedure. Antisera were injected into the hatched area, the dotted line indicating the site of nerve crush. Arrows indicate the two points at which the number of axons containing either CGRP or Reg-2 crossing each point in a longitudinal section were counted. A measure of the number of axons crossing the 3mm and 6mm points was obtained by counting all of the labelled axonal profiles in focus in a single, optimal focal plane in four sections from each nerve. b, reduction in the number of Reg-2 containing axons crossing two points distal to the site of injury following inhibition of Reg-2 activity in vivo. **, p<0.001 at each distance (Student's t-test). c, effects of inhibiting Reg-2 activity on the regeneration of CGRP-containing axons; p=890.2 at each distance.

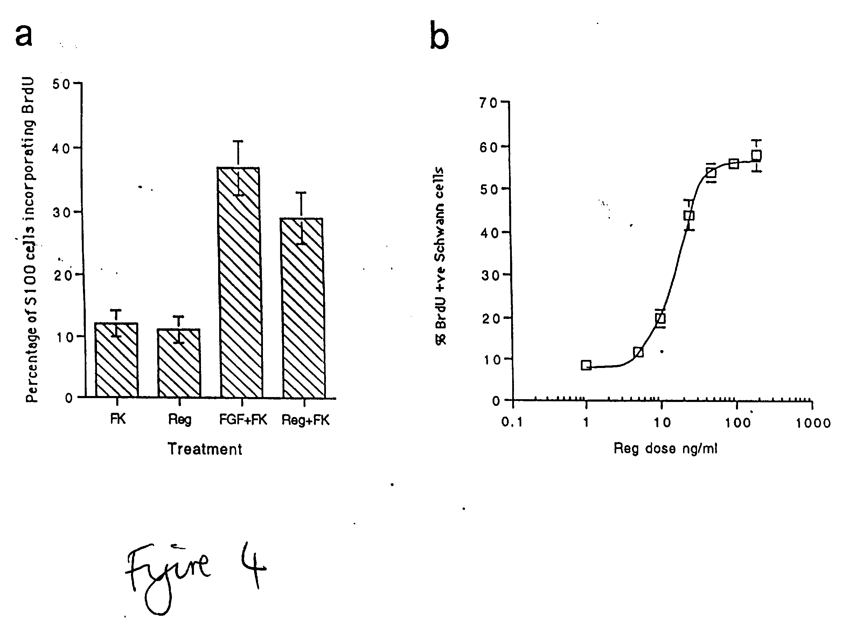

**Figure 4**. Reg-2 is a Schwann cell mitogen *in vitro.* a, graph illustrating the percentage of Schwann cells double-labelled for BrdU incorporation (an indicator of DNA synthesis) and S100 (a Schwann cell marker) in the 24 hours following each treatment. Bars indicate the mean percentage of Schwann cells double-labelled for BrdU averaged over three separate experiments, error bars indicate s.e.m.'s. FK - forskolin; FGF - basic fibroblast growth factor; Reg - recombinant Reg-2 protein. b, dose-response curve for the mitogenic effect of Reg-2 on Schwann cells in the presence of 10 =B5m forskolin. Error bars indicate s.e.m.'s.

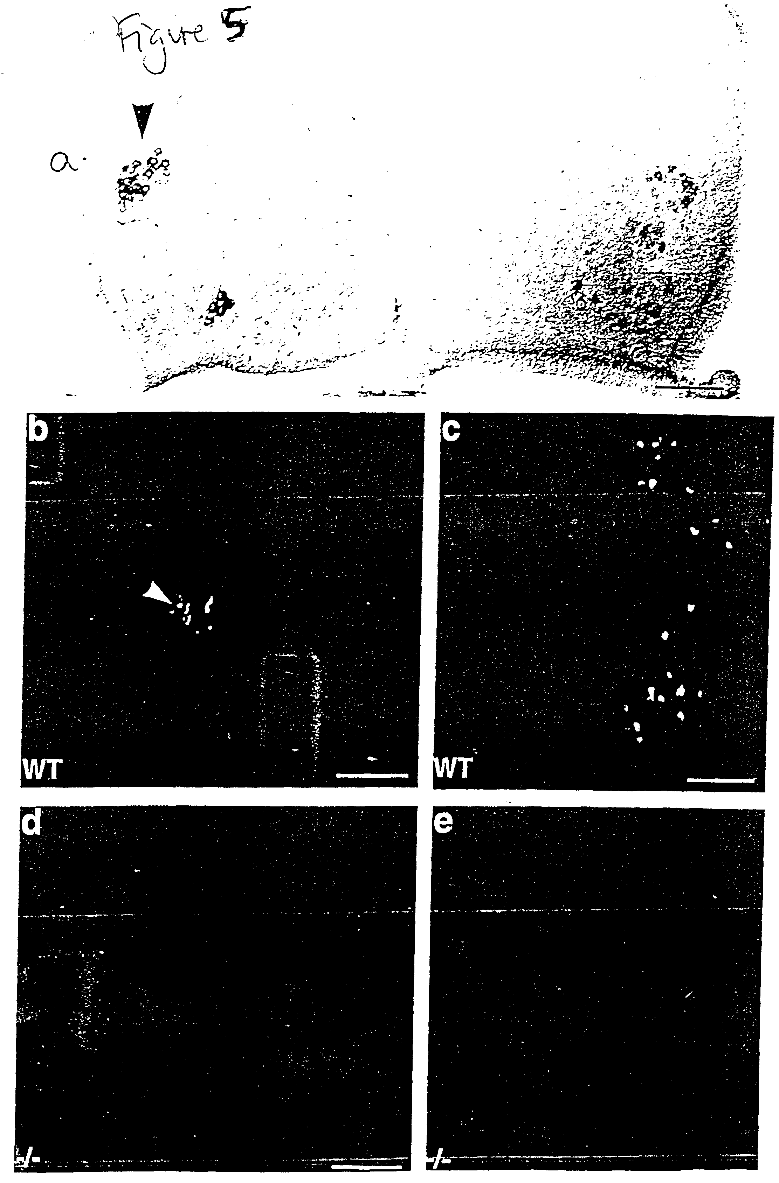

**Figure 5**. Reg-2 is a Schwann cell mitogen *in vitro.* Figure 5a, Reg-2 protein expression in the neonatal spinal cord 24 hours after unilateral sciatic nerve axotomy. Reg-2 is constitutively expressed in the pool of motor neurons projecting into the sciatic nerve (arrowhead). On the side of axotomy, Reg-2 expression has decreased to low levels. b-e, confocal scanning laser photomicrographs of Reg-2 protein expression in the lumbar spinal cords of wild-type (WT) and leukaemia inhibitory factor ("LIF") mutant mouse embryo littermates. b, c Reg-2 in motor neurons (b) and DRG (c) of E15 WT spinal cord. d, e, absence of Reg-2 expression in the spinal cord (d) and DRG (e) of E15 LIFR -/- mouse embryos. Expression was studied in three WT, three LIFR mutant heterozygotes and three LIFR mutant homozygotes. Scale bars, a, 100µm; b & d, 150µm; c & e, 120µm.

### DISCUSSION

We report here on the identification by mRNA differential display³ of an extracellular signalling molecule, previously described as the pancreatic secreted protein Reg-2⁴, which is expressed solely in regenerating and developing rat motor and sensory neurons. Axon-stimulated Schwann cell proliferation is necessary for successful regeneration⁵'⁶, and Reg-2 is a potent Schwann cell mitogen *in vitro. In vivo,* Reg-2 protein is transported along regrowing axons and inhibition of Reg-2 signalling significantly retards the regeneration of Reg-2-containing axons. During development, Reg-2 production by motor and sensory neurons is regulated by contact with peripheral targets. Strong candidates for peripheral factors regulating Reg-2 production are cytokines of the LIF/CNTF family, as Reg-2 is not expressed in developing motor or sensory neurons of mice carrying a targeted disruption of the LIF receptor gene, a common component of the receptor complexes for all of the LIF/CNTF family⁷. Here, CNTF stands for ciliary neurotrophic factor.

Reg-2 is a 16 kD secreted protein which belongs to a family of similar proteins found in the pancreas and gastrointestinal tract under normal and pathological conditions^{4, 8, 9}. A closely related protein, Reg-1, is an islet cell mitogen and is expressed in developing and Alzheimer's disease-affected human cerebral cortex^{10, 11}. Reg-2 is not normally expressed in the adult rat central or peripheral nervous system. Within twenty-four hours of sciatic nerve crush, all motor neurons and a subpopulation of sensory neurons express high levels of Reg-2 mRNA and protein (Figs. 1b & c; 2a & b) and continue to do so for the period of regeneration. The expression of Reg-2 is specific to regenerating motor and sensory neurons, as it is not expressed following a variety of other neuronal lesions, including mechanical and ischaemic cerebral cortex injuries and excitotoxic lesions of the basal ganglia. Nerve crush and injection of the retrograde tracer Fluorogold into the proximal nerve indicated that Reg-2 is expressed by virtually all regenerating sciatic nerve motor neurons (>95%; Fig. 2a & b), but only by a population of medium to large-sized sensory neurons (∼16%; average cell diameter, 39.7±0.8 µm). Retrograde tracing of muscle afferents by injection of Fluorogold into muscle and double-labelling with Reg-2 or double-labelling of sensory neurons for the neuropeptide calcitonin gene-related peptide (CGRP) and Reg-2 three to seven days after sciatic nerve crush indicated that Reg-2 containing sensory neurons do not project to muscle and only a very small number (<4%) co-express CGRP (data not shown). Reg-2 protein is orthogradely transported along growing motor and sensory axons, as demonstrated by the accumulation of Reg-2 protein proximal to the site of a peripheral nerve ligation (Fig. 2c). *In situ* hybridisation with a Reg-2 mRNA specific oligonucleotide probe demonstrated that Reg-2 is not transcribed in the resting or regenerating peripheral nerve. During rat development, Reg-2 is not expressed before embryonic day 15 (Fig. 5b, c). At this stage, Reg-2 protein is expressed by motor and sensory neurons, and no other cell type, and is expressed in some motor and sensory neurons until the fifth postnatal day.

In order to investigate the possible role of this protein in regeneration in vivo, the effect of inhibiting Reg-2 signalling in the sciatic nerve during regeneration was investigated. Reg-2 signalling was inhibited in vivo by intraneural injection of an anti-Reg-2 polyclonal antiserum which blocks the mitogenic effects of Reg-2 in vitro. Four days after nerve crush and antiserum administration, the number of Reg-2-containing axons crossing a point 3 mm from the site of injury was reduced to approximately one-third compared to Reg-2-containing axons in animals treated with pre-immune serum (18.3±1.6 Reg-2 containing axons/section at 3mm distal from the injury site in control animals, compared to 6.3±1 Reg-2 containing axons/section at 3mm distal from the injury site in animals treated with anti-Reg-2 antiserum; see Fig. 3 for details). This effect was specific for Reg-2 containing axons. The regeneration of axons containing the neuropeptide CGRP was slightly reduced in the same series of experiments (from an average of 116±8 axons/section to 103±13 axons/section following Reg-2 inhibition; Fig. 3), although this did not reach significance (p=0.19). However, CGRP-containing axons are a mixture of motor and sensory axons¹², the motor axons of which also express Reg-2, while the majority of CGRP-expressing sensory neurons do not co-express Reg-2 (<4% of CGRP-containing sensory neurons co-express Reg-2).

Given the effects of blocking Reg-2 signalling *in vivo* and the temporal expression of Reg-2 during development, the effect of Reg-2 on Schwann cell proliferation *in vitro* was examined. There is a possibility that recombinant Reg-2 on its own acts as a weak mitogen for Schwann cells in vitro (Fig. 4), as do other known Schwann cell mitogens, such as basic fibroblast growth factor (bFGF) and platelet-derived growth factor (PDGF) ¹³. However, in the presence of forskolin, an activator of adenylate cyclase, Reg-2 is strongly mitogenic for Schwann cells (Fig. 4). This is a well-described phenomenon for other known Schwann cell mitogens, including the glial growth factors (GGFs), bFGF and PDGF, and is attributed to a cAMP-induced increase in receptor expression for those factors ^{6, 14}. To test the hypothesis that the expression of Reg-2 in developing motor neurons is regulated by peripherally-derived factors, the effect of axotomy on Reg-2 expression in the neonatal period was studied. Neonatal rodent motor neurons are developing neurons, which do not assume an adult phenotype until approximately the fifth postnatal day¹⁵. Unlike their adult counterparts, neonatal motor neurons are particularly sensitive to peripheral nerve axotomy, with as many as 90% of lumbar motor neurons projecting into the sciatic nerve undergoing apoptosis in the week following sciatic nerve injury on the first postnatal day¹⁵. This sensitivity to axotomy has been attributed to the interruption of retrograde transport of peripherally-derived neurotrophic factors, as survival of neonatal motor neurons following axotomy can be increased to a varying degree by administration of a range of factors, including BDNF¹⁶ and CNTF¹⁷. Within 24 hours of peripheral nerve axotomy in newborn rat pups, constitutive Reg-2 protein levels in sciatic motor neurons are markedly reduced compared to the uninjured motor neurons of the opposite side of the spinal cord (n=5; Fig. 5a). This reduction in Reg-2 expression precedes motor neuron cell death, approximately half of which occurs in the first four days after axotomy¹⁸. The rapid down-regulation of Reg-2 protein following axotomy in the neonate indicates that Reg-2 expression in developing motor neurons is regulated by contact with peripheral targets. Strong candidates for factors produced by peripheral targets to regulate expression of Reg-2 are cytokines of the LIF/CNTF family. Studies on the control of Reg-2 transcription in the pancreas have identified two interleukin-6 response elements (IL-6REs; STAT-binding elements¹⁹) in the promoter region of the rat Reg-2 gene²⁰. Interleukin-6-related neurotrophic cytokines of the LIF/CNTF family, including LIF, CNTF and cardiotrophin-1 ("CT-1"), are expressed in the developing and adult peripheral tissues^{17, 21, 22}. All of the members of this sub-family of cytokines signal through receptor complexes containing the common transmembrane units, the IL-6 receptor gp130 and LIFR, and activate the intracellular Jak-STAT signalling pathway^{19, 23}. Targeted disruption of the LIFR gene removes a common component of the LIF, CNTF and CT-1 receptors and thus inhibits signalling by all of those factors during development⁷. We analysed E15 mouse embryos homozygous for a disrupted LIFR gene⁷ and found that Reg-2 is not expressed by developing motor and sensory neurons in mutant animals (Fig. 5b-e). Reg-2 is expressed in both of those cell types in both wild type littermates and embryos heterozygous for the disrupted gene (Fig. 5). Thus, Reg-2 expression in developing motor and sensory neurons may be dependent on cytokines of the LIF family acting through a receptor complex containing LIFR.

As mentioned above, Schwann cell dedifferentiation and proliferation are essential for neural regeneration in the adult peripheral nervous system^{5, 6}. The classic Schwann cell mitogens, the glial growth factors/neuregulins are consitutively expressed by motor and sensory neurons in early development and adulthood²⁴ but are not upregulated during regeneration²⁵. In contrast, we have found that endogenous Reg-2 is only produced during periods of Schwann cell proliferation, that is in the perinatal period and during regeneration. We report here that this Schwann cell mitogen is necessary for regeneration of Reg-2 containing axons, a large proportion of which are motor axons. Given the regulation of Reg-2 production during development by peripherally-derived factors, including cytokines of the LIF/CNTF family, it is proposed that Reg-2 is an essential component of the neuron-glia interactions underlying development and regeneration of mammalian motor neurons.

### Sequence homology

As indicated above, the present invention concerns the sequence presented as SEQ ID No. 1 or a variant, derivative or homologue thereof in the manufacture of a medicament to act as a Schwann cell mitogen.

Preferably, the variant, derivative or homologue can have at least 75% sequence homology (i.e. identity) with the sequence presented as SEQ ID No. 1.

In particular, the term "homology" as used herein may be equated with the term "identity".

Here, sequence homology can be determined by a simple "eyeball" comparison of SEQ ID No. 1 with another sequence to see if that other sequence has at least 75% identity to SEQ ID No. 1.

Relative sequence homology (i.e. sequence identity) can also be determined by commercially available computer programs that can calculate % homology between two or more sequences. A typical example of such a computer program is CLUSTAL.

Sequence homology (or identity) may moreover be determined using any suitable homology algorithm, using for example default parameters. Advantageously, the BLAST algorithm is employed, with parameters set to default values. The BLAST algorithm is described in detail at http://www.ncbi.nih.gov/BLAST/blast_help.html, which is incorporated herein by reference. The search parameters are defined as follows, and are advantageously set to the defined default parameters.

Advantageously, "substantial homology" when assessed by BLAST equates to sequences which match with an EXPECT value of at least about 7, preferably at least about 9 and most preferably 10 or more. The default threshold for EXPECT in BLAST searching is usually 10.

BLAST (Basic Local Alignment Search Tool) is the heuristic search algorithm employed by the programs blastp, blastn, blastx, tblastn, and tblastx; these programs ascribe significance to their findings using the statistical methods of Karlin and Altschul (see http://www.ncbi.nih.gov/BLAST/blast_help.html) with a few enhancements. The BLAST programs were tailored for sequence similarity searching, for example to identify homologues to a query sequence. The programs are not generally useful for motif-style searching. For a discussion of basic issues in similarity searching of sequence databases, see Altschul *et al* (1994) Nature Genetics 6:119-129.

The five BLAST programs available at http://www.ncbi.nlm.nih.gov perform the following tasks:
**blastp** compares an amino acid query sequence against a protein sequence database;
**blastn** compares a nucleotide query sequence against a nucleotide sequence database;
**blastx** compares the six-frame conceptual translation products of a nucleotide query sequence (both strands) against a protein sequence database;
**tblastn** compares a protein query sequence against a nucleotide sequence database dynamically translated in all six reading frames (both strands).
**tblastx** compares the six-frame translations of a nucleotide query sequence against the six-frame translations of a nucleotide sequence database.

BLAST uses the following search parameters:

HISTOGRAM Display a histogram of scores for each search; default is yes. (See parameter H in the BLAST Manual).

DESCRIPTIONS Restricts the number of short descriptions of matching sequences reported to the number specified; default limit is 100 descriptions. (See parameter V in the manual page). See also EXPECT and CUTOFF.

ALIGNMENTS Restricts database sequences to the number specified for which high-scoring segment pairs (HSPs) are reported; the default limit is 50. If more database sequences than this happen to satisfy the statistical significance threshold for reporting (see EXPECT and CUTOFF below), only the matches ascribed the greatest statistical significance are reported. (See parameter B in the BLAST Manual).

EXPECT The statistical significance threshold for reporting matches against database sequences; the default value is 10, such that 10 matches are expected to be found merely by chance, according to the stochastic model of Karlin and Altschul (1990). If the statistical significance ascribed to a match is greater than the EXPECT threshold, the match will not be reported. Lower EXPECT thresholds are more stringent, leading to fewer chance matches being reported. Fractional values are acceptable. (See parameter E in the BLAST Manual).

CUTOFF Cutoff score for reporting high-scoring segment pairs. The default value is calculated from the EXPECT value (see above). HSPs are reported for a database sequence only if the statistical significance ascribed to them is at least as high as would be ascribed to a lone HSP having a score equal to the CUTOFF value. Higher CUTOFF values are more stringent, leading to fewer chance matches being reported. (See parameter S in the BLAST Manual). Typically, significance thresholds can be more intuitively managed using EXPECT.

MATRIX Specify an alternate scoring matrix for BLASTP, BLASTX, TBLASTN and TBLASTX. The default matrix is BLOSUM62 (Henikoff & Henikoff, 1992). The valid alternative choices include: PAM40, PAM120, PAM250 and IDENTITY. No alternate scoring matrices are available for BLASTN; specifying the MATRIX directive in BLASTN requests returns an error response.

STRAND Restrict a TBLASTN search to just the top or bottom strand of the database sequences; or restrict a BLASTN, BLASTX or TBLASTX search to just reading frames on the top or bottom strand of the query sequence.

FILTER Mask off segments of the query sequence that have low compositional complexity, as determined by the SEG program of Wootton & Federhen (1993) Computers and Chemistry 17:149-163, or segments consisting of short-periodicity internal repeats, as determined by the XNU program of Claverie & States (1993) Computers and Chemistry 17:191-201, or, for BLASTN, by the DUST program of Tatusov and Lipman (see http://www.ncbi.nlm.nih.gov). Filtering can eliminate statistically significant but biologically uninteresting reports from the blast output (e.g., hits against common acidic-, basic- or proline-rich regions), leaving the more biologically interesting regions of the query sequence available for specific matching against database sequences.

Low complexity sequence found by a filter program is substituted using the letter "N" in nucleotide sequence (e.g., "NNNNNNNNNNNNN") and the letter "X" in protein sequences (e.g., "XXXXXXXXX").

Filtering is only applied to the query sequence (or its translation products), not to database sequences. Default filtering is DUST for BLASTN, SEG for other programs.

It is not unusual for nothing at all to be masked by SEG, XNU, or both, when applied to sequences in SWISS-PROT, so filtering should not be expected to always yield an effect. Furthermore, in some cases, sequences are masked in their entirety, indicating that the statistical significance of any matches reported against the unfiltered query sequence should be suspect.

NCBI-gi Causes NCBI gi identifiers to be shown in the output, in addition to the accession and/or locus name.

Most preferably, sequence comparisons are conducted using the simple BLAST search algorithm provided at http://www.ncbi.nlm.nib.gov/BLAST.

Other computer program methods to determine identify and similarity between the two sequences include but are not limited to the GCG program package (Devereux *et al* 1984 Nucleic Acids Research 12: 387and FASTA (Atschul *et al* 1990 J Molec Biol 403-410).

### Medicaments

As indicated above, the present invention concerns the sequence presented as SEQ ID No. 1 or a variant, derivative or homologue thereof in the manufacture of a medicament to act as a Schwann cell mitogen.

Here, the pharmaceutical compositions may comprise one or more of a pharmaceutically acceptable diluent, carrier, excipient or adjuvant (including combinations thereof).

The pharmaceutical compositions may be for human or animal usage and will typically comprise any one or more of a pharmaceutically acceptable diluent, carrier, excipient or adjuvant. The choice of pharmaceutical carrier, excipient or diluent can be selected with regard to the intended route of administration and standard pharmaceutical practice. The pharmaceutical compositions may comprise as - or in addition to - the carrier, excipient or diluent any suitable binder(s), lubricant(s), suspending agent(s), coating agent(s), solubilising agent(s).

Where appropriate, the pharmaceutical compositions can be administered by inhalation, in the form of a suppository or pessary, topically in the form of a lotion, solution, cream, ointment or dusting powder, by use of a skin patch, orally in the form of tablets containing excipients such as starch or lactose, or in capsules or ovules either alone or in admixture with excipients, or in the form of elixirs, solutions or suspensions containing flavouring or colouring agents, or they can be injected parenterally, for example intracavernosally, intravenously, intramuscularly or subcutaneously. For parenteral administration, the compositions may be best used in the form of a sterile aqueous solution which may contain other substances, for example enough salts or monosaccharides to make the solution isotonic with blood. For buccal or sublingual administration the compositions may be administered in the form of tablets or lozenges which can be formulated in a conventional manner.

### Manufacture

The protein of the present invention may be made by synthetic chemical reaction(s) and/or by use of recombinant DNA techniques.

All publications mentioned in the above specification are herein incorporated by reference. Various modifications and variations of the described methods and system of the invention will be apparent to those skilled in the art without departing from the scope and spirit of the invention. Although the invention has been described in connection with specific preferred embodiments, it should be understood that the invention as claimed should not be unduly limited to such specific embodiments. Indeed, various modifications of the described modes for carrying out the invention which are obvious to those skilled in molecular biology or related fields are intended to be within the scope of the following claims.

### REFERENCES

1. Fawcett, J.W. & Keynes, R.J. Peripheral nerve regeneration. Ann. Rev. Neurosci., 13, 43-60 (1990).
2. Scherer, S.S. & Salzer, J.L. Axon-Schwann cell interactions during peripheral nerve degeneration and regeneration, in Glial Cell Development (eds. Jessen, K.R. & Richardson, W.D.) BIOS, Oxford, (1996).
3. Liang, P., et al. Differential display using one-base anchored oligo-dT primers. Nuc. Acids Res., 22, 5763-5764 (1994).
4. Iovanna, J., Orelle, B., Keim, V. & Dagorn, J.-C. Messenger RNA sequence and expression of rat pancreatitis-associated protein , a lectin-related protein overexpressed during acute experimental pancreatitis. J. Biol. Chem., 266, 24664-24669 (1991).
5. Hall, S.M. The effect of inhibiting Schwann cell mitosis on the re-innervation of acellular autografts in the peripheral nervous system of the mouse. Neuropath. Appl. Neurobiol., 12, 401-414 (1986).
6. Reynolds, M.L. & Woolf, C.J. Reciprocal Schwann cell-axon interactions. Curr. Opin. Neurobiol., 3, 683-693 (1993).
7. Li, M., Sendtner, M. & Smith, A. Essential function of LIF receptor in motor neurons. Nature, 378, 724-727 (1995).
8. Frigerio, J.-M., Dusetti, N.J., Keim, V., Dagorn, J.-C. & Iovanna, J.L. Identification of a second rat pancreatitis-associated protein. Messenger RNA cloning, gene structure, and expression during acute pancreatitis. Biochemistry, 32, 9236-9241 (1993).
9. Frigerio, J.M., Dusetti, N.J., Garrido, P., Dagorn, J.-C. & Iovanna, J.-L. The pancreatitis-associated protein-III (PAP-III), a new member of the PAP gene family. Biochim. Biophys. Acta, 1216, 329-331 (1993).
10. Watanabe, T., et al. Pancreatic beta-cell replication and amelioration of surgical diabetes by Reg protein. Proc. Natl. Acad. Sci. USA, 91, 3589-3592 (1994).
11. de la Monte, S.M., Ozturk, M. & Wands, J.R. Enhanced expression of an exocrine pancreatic protein in Alzheimer's disease and the developing human brain. J. Clin. Invest., 86, 1004-1013 (1990).
12. Dumoulin, F.L., Raivich, G., Streut, W.J. & Kreutzberg, G.W. Differential regulation of calcitonin gene-related peptide (CGRP) in regenerating rat facial nucleus and dorsal root ganglion. Eur. J. Neurosci., 3, 338-342 (1991).
13. Eccleston, P.A. Regulation of Schwann cell proliferation: mechanisms involved in peripheral nerve development. Exp. Cell Res., 199, 1-9 (1992).
14. Weinmaster, G. & Lemke, G. Cell-specific cyclic AMP-mediated induction of the PDGF receptor. EMBO J, 9, 915-920 (1990).
15. Greensmith, L. & Vrbov=El, G. Motoneurone survival: a functional approach. Trends Neurosci., 19, 450-455 (1996).
16. Yan, Q., Elliott, J. & Snider, W.D. Brain-derived neurotrophic factor rescues spinal motor neurons from axotomy-induced cell death. Nature, 360, 753-759 (1992).
17. Sendtner, M., Kreutzberg, G.W. & Thoenen, H. Ciliary neurotrophic factor prevents the degeneration of motor neurons after axotomy. Nature, 345, 440-441 (1990).
18. Schmalbruch, H. & Rosenthal, A. Neurotrophin-4/5 postpones the death of injured spinal motoneurons in newborn rats. Brain=20Res., 700, 254-260 (1995).
19. Schindler, C. & Darnell Jr., J.E. Transcriptional responses to polypeptide ligands: the JAK-STAT pathway. Ann. Rev. Biochem., 64, 621-651 (1995).
20. Dusetti, N.J., Ortiz, E.M., Mallo, G.V., Dagorn, J.-C. & Iovanna, J.L. Pancreatitis-associated protein (PAP I), an acute phase protein induced by cytokines. J. Biol. Chem., 270, 22417-22421 (1995).
21. Banner, L.R. & Patterson, P.H. Major changes in the expression of the mRNAs for cholinergic differentiation factor/leukaemia inhibitory factor and its receptor after injury to adult peripheral nerves and ganglia. Proc.Natl. Acad. Sci. USA, 91, 7109-7113 (1994).
22. Pennica, D., et al. Cardiotrophin-1, a cytokine present in embryonic muscle, supports long-term survival of spinal motoneurons. Neuron, 17, 63-74 (1996).
23. Stahl, N., et al. Association and activation of Jak-Tyk kinases by CNTF-LIF-OSM-IL-6 b receptor components. Science, 263, 92-95 (1994).
24. Gassmann, M. & Lemke, G. Neuregulins and neuregulin receptors in neural development. Curr. Opin. Neurobiol., 7, 87-92 (1997).
25. Carroll, S.L., Miller, M.L., Frohnert, P.W., Kim, S.S. & Corbett, J.A. Expression of neuregulins and their putative receptors, ErbB2 and ErbB3, is induced during Wallerian degeneration. J. Neurosci., 17, 1642-1659 (1997).
26. Jenkins, R. & Hunt, S.P. Long term increases in the levels of c-jun mRNA and Jun protein like immunoreactivity in motor and sensory neurons following axon damage. Neurosci. Lett., 129, 107-111 (1991).
27. Altschul, S.F., Gish, W., Miller, W., Myers, E.W. & Lipman, D.J. Basic local alignment search tool. J. Mol. Biol., 215, 403-410 (1990).
28. Wisden, W., Morris, B.J. & Hunt, S.P. In situ hybridization with synthetic DNA probes, in Molecular Neurobiology. A Practical Approach (eds. Chad, J. & Wheal, H.) IRL Press, Oxford, (1991).
29. De Felipe, C. & Hunt, S.P. The differential control of c-jun expression in regenerating sensory neurons and their associated glial cells. J. Neurosci., 14, 2911-2923 (1994).
30. Chakraborty, C., et al. Plasma-clearance, tissue uptake and expression of pituitary peptide 23/pancreatitis-associated protein in the rat. J. Endocrinol., 145, 461-469 (1995).

SEQUENCES

SEQ I.D. No.1

Protein:

MLHRLAFPVMSWMLLSCLMLLSQVQGEDSPKKIPSARISC

PKGSQAYGSYCYALFQIPQTWFDAELACQKRPEGHLVSVL

NVAEASFLASMVKNTGNSYQYTWIGLHDPTLGGEPNGGGW

EWSNNDIMNYVNWERNPSTALDRGFCGSLSRSSGFLRWRD

TTCEVKLPYVCKFTG

SEQ ID No.2

Nucleotide Sequence:

1 aaaaccatcc aaatcgcccg caagacagct aaggaggagc agaaagatga tgagagttaa

61 tatgttgcat cgcttggcct tcccagtcat gtcctggatg ctgctctcct gcctgatgct

121 cttatcacag gtgcaaggag aagactctcc gaagaaaata ccctctgcac gcattagttg

181 ccccaaaggc tcccaggcat atggctccta ctgctatgcc ctgtttcaga taccacagac

241 ctggtttgat gcagaactgg cctgccagaa gagacctgaa ggacaccttg tatctgtgct

301 caatgtagct gaagcttcat tcttggcatc catggtcaag aacactggaa acagctacca

361 atatacctgg attggactcc atgaccccac tcttggtgga gaacccaatg gaggtggatg

421 ggagtggagt aacaatgaca taatgaatta tgtcaactgg gagaggaacc catctactgc

481 cttagaccgc ggattctgtg gcagcttgtc aagatcttct ggatttctaa gatggagaga

541 taccacatgt gaagtgaagt tgccctacgt ctgcaaattt acaggttaaa attaccagac

601 agcaaacagc tttagtttgt cctgaagcac atcctgtcaa ggggcaaaat atgaagactt

661 gcgtagaaaa agtgtattct atctacagtc catattggag ctctaatcat tctttagcca

721 attttgtata agttgtgtcc tcatgtcttg gaaagcagta ataaacctca gtctctcttc

781 g

## Claims

1. Use of a protein comprising the sequence presented as SEQ ID No. 1 or a variant, derivative or homologue thereof in the manufacture of a medicament to act as a Schwann cell mitogen.

2. A medicament comprising a protein comprising the sequence presented as SEQ ID No. 1 or a variant, derivative or homologue thereof optionally admixed with a pharmaceutically acceptable carrier, diluent or excipient.

3. A medicament comprising a protein comprising the sequence presented as SEQ ID No. 1 or a variant, derivative or homologue thereof optionally admixed with a pharmaceutically acceptable carrier, diluent or excipient, and wherein the protein is in an effective amount to act as a Schwann cell mitogen.

4. A method of treatment comprising administering to a subject a protein comprising the sequence presented as SEQ ID No. 1 or a variant, derivative or homologue thereof and wherein the protein affects the development (e.g. repair and/or regeneration) of neuronal cell or tissue.

5. A method of treatment comprising administering to a subject a protein comprising the sequence presented as SEQ ID No. 1 or a variant, derivative or homologue thereof and wherein the protein acts as a Schwann cell mitogen.

6. A method of treatment comprising administering to a subject a protein comprising the sequence presented as SEQ ID No. 1 or a variant, derivative or homologue thereof and wherein the protein is in an amount to cause regeneration of at least some neuronal cells or tissue.

7. A process of repairing and/or regenerating a neuronal cell or tissue, the process comprising contacting a damaged neuronal cell or tissue with a protein comprising the sequence presented as SEQ ID No. 1 or a variant, derivative or homologue thereof, and allowing repair and/or regeneration of at least some of the neuronal cell or tissue.

8. A Schwann cell mitogen that is capable of being upregulated during regeneration of neuronal cells or tissue.

9. A use, medicament, method or process substantially as described herein.
